# EUROPÄISCHE PATENTSCHRIFT

(11) **EP 0 409 364 B1**
(45) Veröffentlichungstag und Bekanntmachung des Hinweises auf die Patenterteilung: **28.09.1994**
(21) Anmeldenummer: 90250174.1
(22) Anmeldetag: 05.07.1990
(51) Int. Cl.: A61B 17/58

(54) **Verbindungselement für die Osteosynthese**
Junction element for osteosynthesis
Elément de jonction pour l'osthéosynthèse

(30) Priorität: 13.07.1989 DE 3923411
(43) Veröffentlichungstag der Anmeldung: 23.01.1991
(73) Patentinhaber: ARTOS Medizinische Produkte GmbH, 12277 Berlin (DE)
(72) Erfinder: Kranz, Curt, Dr.-Ing., D-1000 Berlin 62 (DE); Schmitz, Herman-Josef, Dr. Dr., D-1000 Berlin 41 (DE)
(74) Vertreter: Christiansen, Henning, Dipl.-Ing.

(56) Entgegenhaltungen:
- EP-A- 0 270 704
- EP-A- 0 340 159
- DE-U- 7 503 861
- DE-U- 8 133 499
- US-A- 3 779 495

## Beschreibung

Die Erfindung betrifft ein Verbindungselement für die Osteosynthese der im Oberbegriff des Anspruchs 1 angegebenen Art.

Die Vereinigung reponierter Knochenfragmente wird bisher hauptsächlich mittels Schrauben, Platten, äußerer Festhalter, Zuggurtung und Marknagelung durchgeführt.

Die Wirkungsweise von Platten und äußeren Festhaltern entspricht dabei einer Weiterentwicklung bzw. Modifizierung der direkten Verschraubung. Nachteilig bei der Verschraubung reponierter Knochenfragmente ist vor allem eine verbleibende Unsicherheit in bezug auf die Stabilität der Verbindung. Um bestmögliche Stabilität zu erreichen, ist eine genaueste Berücksichtigung aller für den jeweiligen Anwendungsfall charakteristischen Randbedingungen erforderlich. Entsprechend groß ist die Sortimentsbreite bei Schrauben, Platten und äußeren Festhaltern. Darüberhinaus führt die Verwendung von Platten und äußeren Festhaltern zu einer starken Belastung des Gewebes beiderseits der Fraktur, da jeweils mehrere Schrauben zum Einsatz kommen. Die Vorarbeiten vor dem Eindrehen der Schrauben sind sehr zeit- und arbeitsaufwendig.

Das Einsatzgebiet der Zuggurtungsosteosynthese ist eng begrenzt, da die Zuggurtung nur bei einfachen Brüchen oder als Zusatzsicherung neben anderen Methoden anwendbar ist.

Die Vereinigung von Knochenbruchstücken mittels Marknagelung ist ebenfalls auf bestimmte Arten von Brüchen beschränkt. Eine stabile Nagelung ist nur nach Aufbohren der Markhöhle möglich. Um Bewegungen im Frakturspalt zu verhindern, muß der Nagel außerordentlich lang und dick sein. Nagelabmessungen in der Größenordnung von 13 mm Durchmesser und 300 mm Länge sind die Folge. Die Vorbereitung der Marknagelung ist nicht weniger aufwendig als die der Verschraubung.

Der nächste Stand der Technik ist aus EP-A-270704 bekannt, wo ein Befestigungselement zur Festlegung einer Osteosyntheseplatte an einem Knochen beschrieben wird, das aus einer elastisch verformbaren hohlen Hülse aus resorbierbarem Material besteht, die durch Aufspreizen im Knochen verankert wird.

EP-A-340 159, ein Dokument das in Hinblick auf die Benannten Vertragsstaaten DE ES FR GB unter Art. 54(3) fällt, bezieht sich auf ein Verbindungselement für die Osteosynthese, bestehend aus
einer aufspreizbaren, elastisch verformbaren hohlen Hülse mit von einer Stirnfläche aus abnehmendem Innendurchmesser und
einem Stift, dessen Außendurchmesser größer als der kleinste und kleiner als der größte Innendurchmesser der Hülse ist und dessen Länge im wesentlichen der Länge der inneren Bohrung der Hülse entspricht.

Der wesentliche Nachteil aller bekannten Implantate zur osteosynthetischen Vereinigung reponierter Knochenfragmente besteht darin, daß ihre stabilisierende Wirkung relativ gering und schwer voraussagbar ist.

Der Erfindung liegt die Aufgabe zugrunde, ein Verbindungselement zur Vereinigung reponierter Knochenfragmente anzugeben, das sich durch verbesserten Halt und möglichst geringe Belastung des den Bruchspalt umgebenden Gewebes auszeichnet. Außerdem soll eine Nachoperation entbehrlich sein.

Diese Aufgabe wird mit den kennzeichnenden Merkmalen des Anspruchs 1 gelöst.

Die Erfindung basiert auf der Erkenntnis, daß mit Hilfe eines zweiteiligen Implantats, wobei der eine Teil zum Aufspreizen des anderen Teiles dient, ein sehr intensiver Kontakt mit der Knochensubstanz und damit ein fester Sitz des Implantates erzielbar ist. Dabei besteht das Implantat aus einem zylinderähnlichen Aufnahmeteil, das mit einer konischen axialen Bohrung versehen ist und einem Nagel, dessen Schaftdurchmesser zwischen dem kleinsten und dem größten Bohrungsdurchmesser des Aufnahmeteiles liegt und dessen Schaftlänge etwa der der Bohrung des Aufnahmeteiles entspricht. Der Nagel wird nach dem Einsetzen des Aufnahmeteiles zwischen den Knochenfragmenten in die konische Bohrung des Aufnahmeteiles eingeschlagen, womit - geringe Elastizität oder Biegsamkeit des Aufnahmeteiles vorausgesetzt - eine Verkeilung zwischen dem Aufnahmeteil und dem umgebenden Knochengewebe hervorgerufen wird. Durch resorbierenden Abbau des Implantats und Ersatz durch Knochengewebe wird dessen spätere operative Entfernung entbehrlich.

Verstärkt wird dieser Effekt entsprechend einer vorteilhaften Weiterbildung der Erfindung durch Schlitzung der Wandung des Aufnahmeteiles in dem Bereich ungefährer Übereinstimmung des Durchmessers der konischen Bohrung und des Nageldurchmessers. Auf diese Weise läßt sich ein Auseinanderspreizen eines bestimmten Teiles, vorzugsweise des distalen Endes, des Aufnahmeteiles erreichen.

Ist die äußere Mantelfläche des Aufnahmeteiles mit quer zur Bohrung verlaufenden zahnähnlichen Vorsprüngen versehen, resultiert daraus ein widerhakenähnliches Einkrallen des Implantates in das Knochengewebe, wodurch die Wahrscheinlichkeit des unbeabsichtigten Auslockerns des Implantates weiter absinkt. Derartige Vorsprünge können beispielsweise als konzentrische Ringe oder als Gewinde ausgebildet sein.

Nutenartige Vorsprünge, die sich längs der Bohrungsachse erstrecken, verhindern ein unbeabsichtigtes Verdrehen des Implantates.

Das Einschlagen des Nagels kann bei gleichzeitiger Reduzierung der Verkantungsgefahr durch eine stetige Abnahme des Nageldurchmessers in Richtung zur Nagelspitze erleichtert werden. Jedoch muß die Durchmesserabnahme des Nagels geringer als die der Bohrung des Aufnahmeteiles sein.

Die Bohrung des Aufnahmeteiles muß nicht notwendig dessen gesamte Länge durchsetzen. Das kann insbesondere im Hinblick auf die eventuelle Notwendigkeit der Wiederentfernung des Implantates vorteilhaft sein. Bei vollständig durchgehender Bohrung bildet sich nach dem Einsetzen des Implantates am distalen Ende des Aufnahmeteiles ein Hohlraum - vorausgesetzt, der in das Aufnahmeteil eingeschlagende Nagel schließt distal nicht genau mit dem Aufnahmeteil ab. In diesen Hohlraum wächst Knochensubstanz hinein und da die Form des Hohlraumes im allgemeinen die eines Kegelstumpfes mit nach distal abnehmendem Durchmesser ist, hält der eingewachsene Knochen das Implantat bei der Explantation fest. Distal geschlossene Aufnahmeteile müssen materialbedingt so weit nachgiebig sein, daß sie sich beim Einschlagen des Nagels bauchig verformen oder durch Schlitzungen aufspreizen, wodurch allerdings wiederum das Problem des Hohlraumes auftritt.

Soll die Implantationstiefe des Aufnahmeteiles von der Knochenoberfläche ausgehen, kann das Aufnahmeteil zur weiteren Verbesserung des beabsichtigten Sitzes in den Knochenfragmenten mit einem radial nach außen abstehenden kragenähnlichen Anschlag- oder Auflageflansch versehen sein.

Durch einen Rastmechanismus zwischen dem Aufnahmeteil und dem Nagel, ergibt sich nochmals eine Verbesserung der Verankerungssicherheit des Implantates, da auf diese Weise ein nachträgliches Zurückgleiten des unter starkem Druck stehenden Nagels verhindert wird. Beispielsweise kann der Nagel an seinem Kopfende eine konzentrisch um den Schaft angeordnete Rille aufweisen, während die innere Bohrung des Aufnahmeteiles an dem Ende ihres größten Durchmessers mit einer entsprechenden einrastbaren Ringnut versehen ist. Durch diese Begrenzung der Einschlagtiefe des Nagels, ergibt sich auch eine genau definierte Aufspreizung des Aufnahmeteiles, so daß Aussagen zur Wirksamkeit des Implantats möglich sind.

Einer besonders vorteilhaften Weiterbildung der Erfindung gemäß, besteht das gesamte Implantat aus resorbierbarem Material, vorzugsweise aus Polylactid. Damit entfällt das Problem der Entfernung des Implantates, womit auch keine Begrenzung der widerhakenähnlichen Verkrallung mehr notwendig ist. In dieser Ausführung sind die Vorteile der Erfindung voll nutzbar.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine Ausführungsvariante eines erfindungsgemäßen Implantates im Längsschnitt,
Figur 2 eine Ausführungsform eines Aufnahmeteiles im Querschnitt mit Blickrichtung in Implantationsrichtung und
Figur 3 die Darstellung eines Anwendungsfalls im Schnitt.

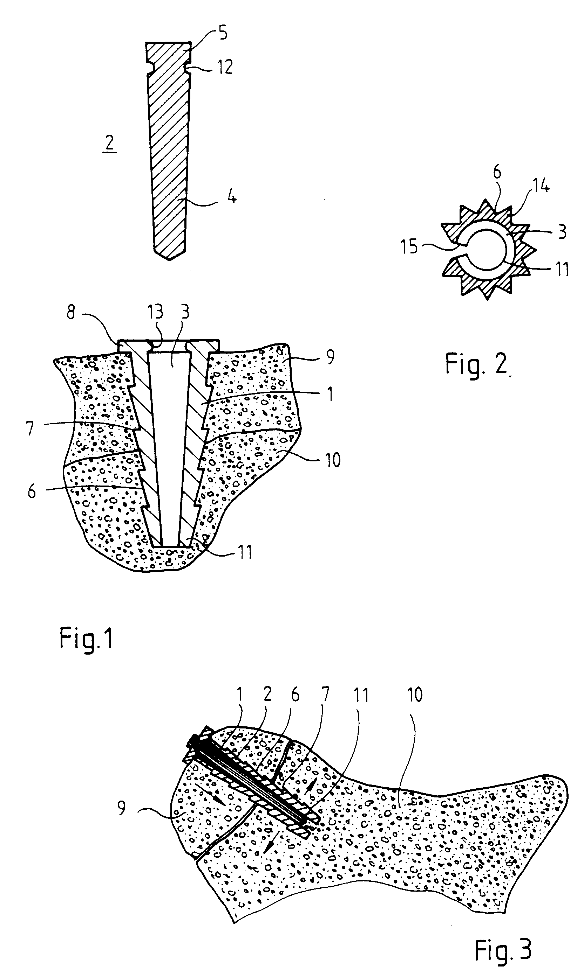

Das erfindungsgemäße Implantat besteht gemäß Figur 1 aus einem Aufnahmeteil 1 und einem Nagel 2. Das Aufnahmeteil 1 ist als zylindrischer Hohlkörper mit einer sich verjüngenden Bohrung 3 ausgebildet. Dabei ist der größte Durchmesser der Bohrung 3 größer und der kleinste Durchmesser der Bohrung 3 kleiner als der Durchmesser des Nagelschaftes 4 über der gesamten Länge des Schaftes 4. Auch der Nagelschaft 4 verjüngt sich ausgehend vom Kopf 5 des Nagels 2. Der Durchmesser der Bohrung 3 nimmt aber über gleiche Distanzen wesentlich stärker ab als der des Nagelschaftes 4. Die Länge der Bohrung 3 entspricht ungefähr der des Nagelschaftes 4. Die äußere Mantelfläche 6 des Aufnahmeteiles 1 ist mit sägezahnähnlichen Vorsprüngen 7 versehen. Die Flanken der Zähne sind widerhakenähnlich zur Implantationsrichtung angeordnet.

Zur Erleichterung der Implantation und einer eventuellen Explantation ist das Aufnahmeteil 1 mit einem Flansch 8 zur Auflage auf den Knochen ausgestattet. Nach dem Einsetzen des Aufnahmeteiles 1 in einer entsprechenden Bohrung, die die zu vereinigenden Knochenbruchstücke 9 und 10 durchsetzt, wird der Nagel 2 in die sich verjüngende Bohrung 3 des Aufnahmeteiles 1 eingeschlagen. Dabei spreizt sich das distale Ende 11 des Aufnahmeteiles 1 auf, wodurch ein widerhakenartiges Verkrallen der sägezahnähnlichen Vorsprünge 7 mit der umgebenden Knochensubstanz des Knochenbruchstückes 10 hervorgerufen wird. Um eine dauerhafte Verbindung zwischen dem Nagel 2 und dem Aufnahmeteil 1 in definierter Relativlage zu garantieren, ist der Nagelschaft 4 mit einer ringförmigen Aussparung 12 versehen, die in einer entsprechenden Ringnut 13 innerhalb der Bohrung 3 des Aufnahmeteiles 1 einrastbar ist.

Die Figur 2 zeigt eine weitere Möglichkeit der Ausführung der Mantelfläche 6. Hierbei sind Längsnute 14 vorgesehen, die in dem der Darstellung entsprechenden Querschnitt sternzackenförmig von der Außenfläche 6 abstehen. Ein Längsschlitz 15 am distalen Ende 11 des Aufnahmeteiles 1 erleichtert das Aufspreizen beim Einschlagen des Nagels 2.

Das zur Vereinigung zweier Knochenbruchstücke 10 und 11 eingesetzte Implantat ist in Figur 3 dargestellt. Die Pfeilrichtungen kennzeichnen die Kraftwirkungen beim Einschlagen des Nagels 2. Es ist ersichtlich, daß das Knochenbruchstück 9 auf das Knochenbruchstück 10 gepreßt wird, wobei gleichzeitig ein Aufspreizen des distalen Endes 11 des Aufnahmeteiles 1 erfolgt. Dadurch verkrallen sich die Vorsprünge 7 der Mantelfläche 6 des Aufnahmeteiles 1 widerhakenartig mit dem Knochenbruchstück 10. Das Implantat besteht vorzugsweise aus resorbierbarem Material, beispielsweise Polylactid, so daß die Notwendigkeit der Entfernung des Implantates nach Heilung des Bruches entfällt. Folglich können die Vorzüge des erfindungsgemäßen Implantates, insbesondere die auslockerungsherabsetzende Wirkung durch das Aufspreizen des Aufnahmeteiles 1 voll ausgeschöpft werden.

## Patentansprüche

1. Verbindungselement für die Osteosynthese,
bestehend aus
einer aufspreizbaren, elastisch verformbaren hohlen Hülse (1) mit von einer Stirnfläche aus abnehmendem Innendurchmesser und einen Stift (2), dessen Außendurchmesser größer als der kleinste und kleiner als der größte Innendurchmesser der Hülse (1) ist und dessen Länge im wesentlichen der Länge der inneren Bohrung (3) der Hülse (1) entspricht, wobei die Hülse und der Stift aus resorbierbarem Material bestehen.

2. Verbindungselement nach Anspruch 1 , **dadurch gekennzeichnet,** daß die Hülse (1) und der Stift (2) aus Polylactid bestehen.

3. Verbindungselement nach einem der vorangehenden Ansprüche, **dadurch gekennzeichnet,** daß die äußere Mantelfläche (6) des Aufnahmeteiles (1) widerhakenartige Vorsprünge (7) aufweist.

4. Verbindungselement nach Anspruch 3 , **dadurch gekennzeichnet,** daß die Vorsprünge (7) wendelförmig oder als konzentrische Ringe ausgebildet sind.

5. Verbindungselement nach Anspruch 4 , **dadurch gekennzeichnet,** daß die Außenkonturen der Vorsprünge (7) in Richtung der Bohrungsachse sägezahnförmig ausgebildet sind.

6. Verbindungselement nach einem der vorangehenden Ansprüche, **durch gekennzeichnet,** daß sich die Vorsprünge (7) wulstartig in Längsrichtung erstrecken.

7. Verbindungselement nach Anspruch 6 , **dadurch gekennzeichnet,** daß die Außenkonturen der Vorsprünge quer zur Bohrungsachse sägezahnförmig ausgebildet sind.

8. Verbindungselement nach einem der vorangehenden Ansprüche, **dadurch gekennzeichnet,** daß sich der Schaft (4) des Stiftes (2) zu seiner Spitze hin verjüngt, wobei der Schaftdurchmesser an der Spitze des Stiftes (2) größer als der kleinste Innendurchmesser der Hülse (1) ist.

9. Verbindungselement nach einem der vorangehenden Ansprüche, **dadurch gekennzeichnet,** daß die innere Bohrung (3) der Hülse (1) durchgehend ist.

10. Verbindungselement nach Anspruch 9, **dadurch gekennzeichnet,** daß die Hülse (1) mindestens einen sich zum Ende hin erstreckenden Längsschlitz (15) aufweist.

11. Verbindungselement nach einem der vorangehenden Ansprüche, **dadurch gekennzeichnet,** daß die Hülse (1) an dem Ende mit dem größten Durchmesser der inneren Bohrung (3) einen radial nach außen weisenden kragenähnlichen Anschlag- oder Auflageflansch (8) aufweist.

12. Verbindungselement nach einem der vorangehenden Ansprüche, **dadurch gekennzeichnet,** daß der Schaft (4) des Stiftes (2) eine ringförmige Nut (12) und die innere Bohrung (3) der Hülse (1) eine der Einschlagtiefe des Stiftes (2) in die Hülse (1) angepaßte und in die Nut (12) einrastbare ringförmige Wulst aufweisen oder umgekehrt.

## Claims

1. A connecting element for osteosynthesis, comprising an expandable, elastically deformable, hollow sleeve (1) with an inside diameter decreasing from a front surface and a pin (2), the outside diameter of which is larger than the smallest inside diameter of the sleeve (1) and smaller than the largest inside diameter, and the length of which substantially corresponds to the internal bore (3) of the sleeve (1), the sleeve and pin being made of resorbable material.

2. A connecting element according to claim 1, characterised in that the sleeve (1) and pin (2) are made of polylactide.

3. A connecting element according to either of the preceding claims, characterised in that the outer generated surface (6) of the receiving member (1) has barb-like projections (7).

4. A connecting element according to claim 3, characterised in that the projections (7) are helical or in the form of concentric rings.

5. A connecting element according to claim 4, characterised in that the external contours of the projections (7) are sawtooth-shaped in the direction of the axis of the bore.

6. A connecting element according to any of the preceding claims, characterised in that the projections (7) extend in a bead shape longitudinally.

7. A connecting element according to claim 6, characterised in that the external contours of the projections are sawtooth-shaped transversely of the axis of the bore.

8. A connecting element according to any of the preceding claims, characterised in that the shank (4) of the pin (2) tapers towards its tip, the diameter of the shank at the tip of the pin (2) being larger than the smallest inside diameter of the sleeve (1).

9. A connecting element according to any of the preceding claims, characterised in that the internal bore (3) of the sleeve (1) extends right through it.

10. A connecting element according to claim 9, characterised in that the sleeve (1) has at least one longitudinal slot extending to the end.

11. A connecting element according to any of the preceding claims, characterised in that the sleeve (1) has a collar-like abutting or bearing flange (8) directed radially outwards, at the end with the largest diameter of the internal bore (3).

12. A connecting element according to any of the preceding claims, characterised in that the shank (4) of the pin (2) has an annular groove (12), and the internal bore (3) of the sleeve (1) has an annular bead which is adapted to the depth to which the pin (2) is driven into the sleeve (1) and which can engage in the groove (12), or vice versa.

## Revendications

1. Elément de coaptation pour l'ostéosynthèse, composé d'un manchon creux (1) expansible, déformable élastiquement, dont le diamètre interne diminue à partir d'une face frontale, et d'un goujon (2) dont le diamètre externe est supérieur au plus petit diamètre interne du manchon (1) et inférieur à son plus grand diamètre et dont la longueur est sensiblement égale à la longueur du trou interne (3) du manchon (1), le manchon et le goujon étant en un matériau biodégradable.

2. Elément de coaptation selon la revendication 1, caractérisé en ce que le manchon (1) et le goujon (2) sont en polylactide.

3. Elément de coaptation selon une des revendications précédentes, caractérisé en ce que la surface externe (6) de la pièce de logement (1) présente des saillies (7) de type contre-pointe.

4. Elément de coaptation selon la revendication 3, caractérisé en ce que les saillies (7) sont conçues sous forme de spirales ou d'anneaux concentriques.

5. Elément de coaptation selon la revendication 4, caractérisé en ce que les contours externes des saillies (7) ont la forme de dents de scie dans la direction de l'axe du trou.

6. Elément de coaptation selon une des revendications précédentes, caractérisé en ce que les saillies (7) s'étendent en bourrelets dans la direction longitudinale.

7. Elément de coaptation selon la revendication 6, caractérisé en ce que les contours externes des saillies ont la forme de dents de scie perpendiculaires à l'axe du trou.

8. Elément de coaptation selon l'une des revendications précédentes, caractérisé en ce que la tige (4) du goujon (2) se rétrécit jusqu'à sa pointe, le diamètre de la tige au niveau de la pointe du goujon (2) étant supérieur au plus petit diamètre interne du manchon (1).

9. Elément de coaptation selon une des revendications précédentes, caractérisé en ce que le trou interne (3) du manchon (1) est traversant.

10. Elément de coaptation selon la revendication 9, caractérisé en ce que le manchon (1) présente au moins une fente longitudinale (15) s'étendant vers son extrémité.

11. Elément de coaptation selon une des revendications précédentes, caractérisé en ce que le manchon (1) présente une bride de butée ou d'appui (8) de type collerette, dirigée radialement vers l'extérieur à l'extrémité de plus grand diamètre du trou interne (3).

12. Elément de coaptation selon une des revendications précédentes, caractérisé en ce que la tige (4) du goujon (2) présente une rainure annulaire (12) et le trou interne (3) du manchon (1) un bourrelet annulaire emboîtable dans la rainure (12) et adapté à la profondeur d'enfoncement du goujon (2) dans le manchon (1) ou vice versa.
